# FASCICULE DE BREVET EUROPEEN

(11) **EP 0 748 591 B1**
(45) Date de publication et mention de la délivrance du brevet: **17.10.2001**
(21) Numéro de dépôt: 95201618.6
(22) Date de dépôt: 16.06.1995
(51) Int. Cl.: A23L 1/305, A23J 3/10, A61K 7/16, A23G 3/00

(54) **Caséines micellaires fluorées**
Fluorierte Kasein-Mizellen
Fluorinated micellar casein

(43) Date de publication de la demande: 18.12.1996
(73) Titulaire: SOCIETE DES PRODUITS NESTLE S.A., 1800 Vevey (CH)
(72) Inventeur: Berrocal, Rafael, CH-1806 St-Légier (CH); Neeser, Jean-Richard, CH-1073 Savigny (CH); Tachon, Pierre, CH-1053 Cugy (CH)

(56) Documents cités:
- EP-A- 0 528 458
- WO-A-91/13607
- WO-A-93/03707
- WO-A-94/00146
- GB-A- 2 206 049
- COLLOIDS AND SURFACTANTS, vol. 3, 1994 pages 177-182, G ROLLA AND M. RYKKE 'EVIDENCE FOR PRESENCE OF MICELLE-LIKE PROTEIN GLOBULES IN HUMAN SALIVA'
- MILCHWISSENSCHAFT, vol. 30, no. 11, 1975 pages 674-680 , A.M. KNOOP, K.H. PETERS 'PHOAPHATASEAKTIVITÄT IN SAUERMILCH'

## Description

La présente invention a pour objet la fluoration de la caséine d'un lait, et l'utilisation des caséines fluorées en tant qu'agents destinés à traiter les pathologies dentaires.

### Etat de la technique.

Des laits de vache comprenant des sels de fluor, comme le fluorure de sodium, ont déjà été proposés pour réduire l'incidence des caries dentaires sur la santé humaine (Beddows G. et al., Analyst, 106, 1341-1344, 1981).

D'autres travaux ont par ailleurs montré que le fluorure s'équilibre majoritairement dans un lait de vache sous une forme ionique libre, tandis qu'une partie minoritaire peut être complexée par le calcium du lait. Le fluorure n'est donc pas naturellement complexée aux protéines du lait, notamment à la caséine micellaire (Beddows G. et al., J. Fd Technology, 17, 55-62, 1982).

D'autre part, on sait que dans une solution de caséine micellaire un excès de sels de fluorure est susceptible de diviser la caséine micellaire en ses sous-unités micellaires, par le jeux des forces ioniques excédentaires apportées par le fluorure (Caroll et al., J. Dairy Science, 54, 752, 197).

Enfin, EP604802 montre que la caséine micellaire présente une activité anti-carie dentaire, et EP283675 montre que les kappa-caséinoglycopeptides exposées à la surface des micelles de caséine présentent eux aussi une activité anti-carie et anti-plaque dentaire.

### Résumé de l'invention

La présente invention a pour objet de fournir de nouveaux dérivés de caséines micellaires pouvant être utilisés pour l'hygiène buccale de l'homme ou des animaux.

A cet effet, dans le procédé de préparation de micelles de caséines fluorées selon la présente invention, on ajoute à une solution comprenant des micelles de caséine au moins 100 ppm d'un sel de fluor soluble et l'on isole les caséines micellaires fluorées.

L'invention concerne également les micelles de caséines fluorées, et les compositions alimentaires ou pharmaceutiques destinées à traiter les pathologies dentaires ou dentinaires comprenant une quantité efficace de caséines micellaires fluorées ou de ses sous-unités micellaires.

Enfin, la présente invention concerne aussi l'utilisation de caséines micellaires fluorées ou de ses sous-unités micellaires fluorées pour la préparation d'une composition destinée à traiter les pathologies dentaires ou dentinaires.

L'invention a permis d'une manière surprenante de fabriquer pour la première fois des caséines micellaires fluorées dont la structure micellaire est conservée.

Le fluorure peut donc s'associer à la micelle sans toutefois perturber sa structure ou la détruire. L'invention va donc à l'encontre des connaissances scientifiques reconnues qui montrent que le fluorure à tendance en fonction de sa concentration soit à détruire la micelle, soit à ne quasiment pas se complexer avec les protéines du lait et en particulier la caséine micellaire du lait (Beddows G. et al., J. Fd Technology, 17, 55-62, 1982).

Les caséines micellaires fluorées selon l'invention sont de plus complexées avec une quantité importante de fluorure. On a en effet pu constater que plus de 20%, voire 50% du fluorure libre initial se trouve ainsi complexé à la caséine, ce qui permet d'envisager leur utilisation pour l'hygiène buccale humaine ou animale en tant qu'agent anti-plaque dentaire, anti-carie dentaire, anti-parodontopathie et anti-hyperesthésie dentinaire.

### Description détaillée de l'invention

Pour mettre en oeuvre le présent procédé, on peut utiliser comme solution comprenant de la caséine micellaire un lait provenant d'un animal tel qu'une vache, une chèvre ou une brebis, par exemple, ou une fraction dudit lait comprenant de la caséine micellaire. Toutefois, il est recommandable de s'assurer que les composés importants pour le maintien de la structure micellaire, tel que le citrate par exemple, soient présents dans ladite fraction en des quantités adéquates. Il faut cependant noter que ces composés sont isolés dans la même fraction que celle comprenant la caséine micellaire lorsque l'on microfiltre, ultrafiltre ou diafiltre un lait animal, et que l'on isole le rétentât.

On ajoute ensuite à la solution au moins 100 ppm d'un sel de fluor soluble dans l'eau. Le sel de fluorure soluble peut être choisi parmi le fluorure de sodium et les fluorophosphates de sodium, par exemple. De préférence, on mélange le sel de fluorure à la solution pendant 1 min à 5 h, à une température comprise entre 5 et 70°C de manière à favoriser la complexation du fluor à la caséine micellaire. Pour préparer des caséines fluorées dont la structure micellaire est conservée, on peut ajouter à la solution 100 à 2000 ppm d'un sel de fluorure soluble. Par contre si l'on veut préparer des sous-unités micellaires de caséines fluorées, on peut ajouter à la solution plus de 2000 ppm d'un sel de fluorure soluble, par exemple.

Enfin si l'on a supplémenté un lait animal par un sel de fluor, on peut isoler la caséine fluorée, micellaire ou sous-micellaire, par microfiltration, ultrafiltration et/ou diafiltration dudit lait. De préférence, on microfiltre ce lait sur une membrane minérale présentant une porosité d'environ 0,1-0,2µm. Par contre, si l'on a supplémenté une fraction d'un lait animal par un sel de fluor, on peut ne pas chercher à purifier davantage la caséine fluorée dans la mesure où sa concentration est suffisamment importante.

Toutefois, il est intéressant d'obtenir une poudre comprenant majoritairement de la caséine fluorée (dont le fluor est complexé aux micelles ou aux sous-unités micellaires), en séchant la fraction dans laquelle on a isolé la caséine fluorée, par exemple par atomisation.

La caséine micellaire fluorée selon la présente invention présente une structure spatiale micellaire identique à celle de la caséine micellaire originelle du lait. Les interactions entre la micelle et le fluorure semblent être des interactions ioniques stables, cependant d'autres genres interactions ne sont pas exclus (absorption physique, covalence par exemple).

Les sous-unités de caséines micellaires complexées à du fluorure présentent également une structure intéressante. Ces sous-unités sont formées de caséines agrégées qui sont complexées avec des molécules de fluorure. Ces sous-unités micellaires fluorées présentent également potentiellement une activité anti-carie et anti-plaque dentaire remarquable, car elles comprennent des kappa-caséinoglycopeptide, des caséinophosphopeptides et du fluorure qui sont connus pour avoir une activité anti-carie et anti-plaque dentaire.

La présente invention concerne donc aussi l'utilisation de caséines micellaires fluorées ou de ses sous-unités micellaires pour la préparation d'une composition destinée à traiter la carie ou la plaque dentaire. Les caséines micellaires fluorées et ses sous-unités peuvent être ainsi utilisées dans une méthode de traitement prophylactique et/ou thérapeutique de la plaque dentaire, de la carie dentaire, et des paradontopathies et hyperesthésies dentinaires.

Pour cela, on prépare une composition alimentaire ou pharmaceutique acceptable comprenant une quantité efficace de caséines micellaires fluorées ou de ses sous-unités micellaires, et on l'administre oralement à l'homme ou à un animal.

De telles compositions peuvent être préparées en ajoutant à une composition alimentaire ou pharmaceutique, au cours de leur préparation, au moins 0,1% de la caséine fluorée selon la présente invention, par exemple. On peut aussi envisager d'ajouter directement à une composition alimentaire ou pharmaceutique, ayant une consistance liquide ou du moins pâteuse, au moins 0,1% d'un mélange comprenant une poudre de caséine micellaire et au moins 100 ppm d'un sel de fluorure soluble. Les caséine fluorées selon la présente invention peuvent donc être aussi fabriquées au cours de la fabrication d'un produit alimentaire ou pharmaceutique.

Les compositions selon l'invention sont ainsi des compositions alimentaires ou pharmaceutiques comprenant une quantité efficace de caséines micellaires fluorées ou de ses sous-unités micellaires, c'est à dire une quantité suffisante, généralement de 0,1 à 90% en poids mais de préférence 1 à 20%, pour inhiber l'adhésion des bactéries de la plaque et de la carie dentaire, par exemple des bactéries du genre *Actinomyces naeslundii, Actinomyces viscosus, Streptococcus sanguis, Streptococcus mutons,* et/ou *Streptococcus sobrinus*.

La composition selon l'invention peut comprendre en outre des ingrédients conventionnels qui sont bien connus de l'homme du métier, qui dépendent de la forme et de l'utilisation du produit final. Pour une destination plutôt alimentaire, la composition peut être un article de confiserie, une boisson sucrée ou un lait pouvant être fermenté, par exemple. Pour une application plutôt pharmaceutique, la composition peut être sous la forme d'une crème dentifrice, d'une pâte dentifrice, d'une gomme dentifrice à mâcher, ou d'un bain de bouche, par exemple.

Dans le cas d'une crème ou d'une pâte dentifrice, la composition peut comprendre en outre une phase liquide humectante, un liant ou un épaississant, des particules abrasives, un surfactant et des arômes. La composition peut ainsi comprendre 10 à 85% d'une phase liquide humectante telle qu'un sirop de glycérol, de sorbitol, de propylène glycol, ou de lacticol, 0,1 à 10% d'un liant ou d'un épaississant tel que le sodium carboxymethylcellulose, les gommes de xanthane ou des gels de silice, 0,5 à 5% d'un surfactant qui ne dissocie pas la micelle ou les sous-unités micellaires, et au moins 3% d'agents abrasifs tel que les silices, les carbonates de calcium, le phosphate anhydre dicalcium ou l'hydroxyapatite, par exemple.

Les composition selon l'invention peuvent aussi comprendre d'autres ingrédients optionnels; par exemple des agents anti-plaque et/ou antimicrobien tel que la chlorhexidine, le 2,4,4'-trichloro-2'-hydroxy-diphenyl éther, les composés de zinc, les métaux pyrophosphates alcalins, le fluorure de sodium ou les fluorophosphate de sodium; des édulcorants comme la saccharine; des agents opacifiants comme la le dioxyde de titane; des agents colorants; ou des agents tampons maintenant un pH approprié tel que l'acide benzoïque ou son sel. De préférence, on choisit un tampon ayant un pH supérieur à 5 de façon à éviter une dissociation de la micelle.

Les exemples ci-après sont présentés à titre d'illustration du procédé, de la composition et de l'utilisation selon la présente invention. Dans ceux-ci, les parties et pourcentages sont donnés en poids, sauf indication contraire.

### Exemples 1

On prépare une solution de caséine micellaire en microfiltrant un lait de vache sur une membrane minérale présentant une porosité d'environ 0,2µm, puis on la sèche par atomisation.

Pour connaître la distribution du fluor dans une suspension de caséine micellaire, on prépare une solution aqueuse comprenant 20% en poids de caséine micellaire. Il faut noter que cette suspension comprend pas ailleurs 200 ppm de calcium libre.

On ajoute 58,25mg de fluorure de sodium à 100g de la solution micellaire (soit 600ppm), on agite la solution pendant une heure, puis on l'ultracentrifuge à 100000G pendant 90 min. On rince ensuite les culots de centrifugation à l'eau distillée, on les disperse dans de l'eau, puis on les lyophilise. On mesure alors la quantité de fluor restant dans la micelle par la méthode de Beddows G. et al. (Analyst, 106, 1341-1344, 1981).

Les résultats présentés dans le tableau de l'exemple 2 montrent que 59,13% du fluorure ajouté sous forme de fluorure de sodium est retrouvé en même temps que la micelle lors de l'ultracentrifugation. De plus, la couleur et la consistance du culot de centrifugation sont caractéristiques de la présence de micelles. La structure micellaire peut être confirmée par microscopie électronique. Le fluorure de sodium ne précipitant pas naturellement à 100000G, les résultats tendent donc à montrer que le fluorure se lie à la micelle par un mécanisme pouvant faire intervenir l'absorption physique, l'échange d'ions et/ou la covalence, par exemple.

Ces résultats vont à l'encontre de l'enseignement technique général montrant que plus de 80% du fluorure, voire 95%, reste en solution après ultracentrifugation d'un lait fluoré, et que la complexation du fluor avec les protéines du lait est inhibée par des teneurs en calcium libre supérieures à 10ppm (Beddows G. et al., J. Fd Technology, 17, 55-62, 1982).

### Exemple 2

On ajoute 30mg de fluorophosphate de sodium à 100g de la solution micellaire de l'exemple 1 (soit 300ppm), on agite la solution pendant une heure, puis on l'ultracentrifuge à 100000G pendant 90 min. On rince ensuite les culots de centrifugation à l'eau distillée, on les disperse dans de l'eau, puis on les lyophilise. On mesure alors la quantité de fluor restant dans la micelle par la méthode citée à l'exemple 1.

Le tableau ci-après illustre les résultats expérimentaux des exemples 1 et 2.

| Exemples | 1) Micelle + NaF | 2) Micelle + NaF6F |
|---|---|---|
| Poids de la solution initiale en g de liquide | 100 | 100 |
| Poids de micelle initiale en g de poudre | 10,108 | 10,097 |
| Fluorure ajouté en mg/ 100g de liquide | 26,47 | 20,36 |
| Micelle ultracentrifugée en poudre | 6,66 | 7,12 |
| Fluorure analysé en mg/ 100g de micelle en poudre | 235 | 112 |
| Fluorure calculé en mg/100g de liquide | 15,65 | 7,97 |
| [Fluorure calculé / Fluorure ajouté] % | 59,13 | 39,17 |

Les résultats montrent que 39,13% du fluorure ajouté sous forme de fluorophosphate de sodium est retrouvé en même temps que la micelle lorsque l'on ultracentrifuge la solution fluorée. De plus, la couleur et la consistance du culot de centrifugation sont aussi caractéristiques de la présence de micelles. La structure micellaire est confirmée par microscopie électronique. Le fluorophosphate de sodium ne précipitant pas naturellement à 100000G, les résultats tendent donc à montrer que le fluorure se lie à la micelle par un mécanisme pouvant faire intervenir l'absorption physique, l'échange d'ions et/ou la covalence, par exemple.

### Exemple 3

On prépare une poudre de caséine micellaire fluorée par la méthode décrite aux exemples 1 et 2, à la différence que l'on ajoute à la solution micellaire 1000ppm de fluorure de sodium.

### Exemple 4

On prépare une poudre de caséine micellaire fluorée en ajoutant à un lait pasteurisé de vache 1000 ppm de fluorophosphate de sodium, en homogénéisant le lait à température ambiante pendant 10 min, en ultrafiltrant le lait sur une membrane de porosité de 0,2µm, puis en séchant le rétentat par atomisation.

### Exemple 5

On prépare une poudre de sous-unités de caséine micellaires fluorées de la manière suivante. On microfiltre un lait de vache pasteurisé sur une membrane minérale présentant une porosité d'environ 0,2µm, puis on dilue le rétentât avec de l'eau de manière à obtenir une solution comprenant 20% en poids de caséine micellaire. On ajoute 3000ppm de fluorure de sodium à la solution de caséine micellaire, on homogénéise la solution pendant quelques minutes, puis on la sèche par atomisation. La poudre ainsi obtenue peut être avantageusement utilisée pour la prépration de compositions alimentaires ou pharmaceutiques destinées à traiter les pathologies dentaires.

Pour confirmer la présence de sous-unités micellaires fluorées. On prépare une solution comprenant 20% de poudre de sous-unités micellaires fluorées, puis on la centrifuge à 100000G. La couleur et la consistance du culot de centrifugation sont caractéristiques de la dissociation des micelles en leur sous-unités. Cette structure sous-particulaire peut être confirmée par microscopie électronique. Par ailleurs, on peut rincer les culots de centrifugation à l'eau distillée, les disperser dans de l'eau, les lyophiliser, puis doser leur teneur en fluor. Les résultats montrent que la poudre ainsi obtenue est particulièrement chargée en fluor.

### Exemple 6

On prépare une pâte dentifrice destinée à l'élimination du tartre et à la prophylaxie de la carie dentaire. Cette poudre comprend 5% de carbonate de calcium, 10% d'aérogel de silice (Gasil 23), 40% de sirop de sorbitol, 1% de carboxymethycellulose de sodium, 6,5% d'une poudre de micelle de caséine fluorée obtenue à l'exemple 4, 0,2% de saccharinate de sodium, 1% de dioxyde de titane, 0,04% de Formalin, 1% d'arôme et le reste d'eau.

### Exemple 7

On prépare la même pâte dentifrice que celle décrite à l'exemple 5, à la différence que l'on remplace les 5% d'une poudre de micelle de caséine fluorée par 5% d'une poudre de sous-unités de micelles de caséine obtenue à l'exemple 5.

### Exemple 8

On prépare un bain de bouche destiné à la prophylaxie de la carie dentaire et le traitement des parodontopathies et de l'hyperesthésie dentinaire. Ce bain comprend 10% de poudre de micelle de caséine fluorée obtenue à l'exemple 4, 0,1% d'acide benzoïque, 0,2% de saccharinate de sodium, 0,1% de jaune de quinoline, 0,1% de bleu patenté, 3% d'alcool éthylique à 95%, et le reste d'eau.

## Revendications

1. Procédé de préparation de micelles de caséine fluorées, dans lequel on ajoute à une solution comprenant de la caséine micellaire au moins 100 ppm d'un sel de fluor soluble et l'on isole la caséine micellaire fluorée.

2. Procédé de préparation de micelles selon la revendication 1, dans lequel on prépare un lait comprenant au moins 10% en poids de caséine micellaire, on ajoute 100-2000 ppm d'un sel de fluor soluble choisi notamment parmi le fluorure de sodium et les fluorophosphates de sodium, et l'on isole la caséine micellaire fluorée

3. Procédé de préparation de micelles selon la revendication 1, dans lequel on prépare un lait comprenant au moins 10% en poids de caséine micellaire, on ajoute plus de 2000 ppm d'un sel de fluor soluble choisi notamment parmi le fluorure de sodium et les fluorophosphates de sodium, et l'on isole les sous-unités de caséine micellaire fluorée.

4. Caséine micellaire fluorée.

5. Caséine selon la revendication 4, fluorée par au moins un sel de fluorure soluble choisi parmi le fluorure de sodium et les fluorophosphates de sodium.

6. Composition alimentaire ou pharmaceutique destinée à traiter la carie ou la plaque dentaire comprenant une quantité efficace de caséines micellaires fluorées ou de ses sous-unités micellaires.

7. Utilisation de caséines micellaires fluorées ou de ses sous-unités micellaires pour la préparation d'une composition destinée à traiter la carie ou la plaque dentaire.

## Claims

1. Method for preparing fluorinated casein micelles, wherein at least 100 ppm of a soluble fluorine salt is added to a solution containing the micellar casein and the fluorinated micellar casein is isolated.

2. Method for preparing micelles according to claim 1, wherein a milk is prepared containing at least 10 % by weight of micellar casein, 100-2000 ppm are added of a soluble fluorine salt chosen in particular from sodium fluoride and sodium fluorophosphates, and the fluorinated micellar casein is isolated.

3. Method for preparing micelles according to claim 1, wherein a milk is prepared containing at least 10 % by weight of micellar casein, more than 2000 ppm of a soluble fluorine salt is added chosen in particular from sodium fluoride and sodium fluorophosphates, and the sub-units of fluorinated micellar casein are isolated.

4. Fluorinated micellar casein.

5. Casein according to claim 4, fluorinated by at least one soluble fluorine salt chosen from sodium fluoride and sodium fluorophosphates.

6. Food or pharmaceutical composition intended for the treatment of dental caries or plaque containing an effective quantity of fluorinated micellar caseins or of their micellar sub-units.

7. Use of fluorinated micellar caseins or of their micellar sub-units for the preparation of a composition intended for the treatment of dental caries or plaque.

## Patentansprüche

1. Verfahren zur Herstellung von fluorierten Casein-Micellen, bei dem man einer micelläres Casein enthaltenden Lösung mindestens 100 ppm eines löslichen Fluorsalzes beigibt und das fluorierte micelläre Casein isoliert.

2. Verfahren zur Herstellung von Micellen nach Anspruch 1, bei dem man eine Milch herstellt, die mindestens 10 Gew.-% micelläres Casein enthält, 100-2000 ppm eines löslichen Fluorsalzes beigibt, das insbesondere aus Natriumfluorid und Natriumfluorphosphaten ausgewählt ist, und das fluorierte micelläre Casein isoliert.

3. Verfahren zur Herstellung von Micellen nach Anspruch 1, bei dem man eine Milch herstellt, die mindestens 10 Gew.-% micelläres Casein enthält, mehr als 2000 ppm eines löslichen Fluorsalzes beigibt, das insbesondere aus Natriumfluorid und Natriumfluorphosphaten ausgewählt ist, und die Untereinheiten von fluoriertem micellären Casein isoliert.

4. Fluoriertes micelläres Casein.

5. Casein nach Anspruch 4, das durch mindestens ein lösliches Fluoridsalz fluoriert ist, das aus Natriumfluorid und Natriumfluorphosphaten ausgewählt ist.

6. Nahrungsmittel- oder Arzneimittelzusammensetzung, die für die Behandlung von Karies oder Zahnplaque bestimmt ist und eine wirksame Menge von fluorierten micellären Caseinen oder ihren micellären Untereinheiten enthält.

7. Verwendung von fluorierten micellären Caseinen oder ihren micellären Untereinheiten für die Herstellung einer Zusammensetzung für die Behandlung von Karies oder Zahnplaque.
